# EUROPEAN PATENT APPLICATION

(11) **EP 3 673 912 A1**
(43) Date of publication of application: **01.07.2020**
(21) Application number: 17922675.8
(22) Date of filing: 22.08.2017
(51) Int. Cl.: A61K 36/28, A23L 33/105

(54) **COMPOSITION FOR TREATING BENIGN PROSTATE HYPERPLASIA AND FUNCTIONAL HEALTH FOOD COMPRISING AUCKLANDIAE RADIX EXTRACT AND COSTUNOLIDE AS ACTIVE INGREDIENTS**

(71) Applicant: QUBEST BIO CO., LTD., Giheung-gu, Yongin-si Gyeonggi-do 16950 (KR)
(72) Inventor: KIM, Kyung-Jae, Guri-si Gyeonggi-do 11947 (KR); SONG, Young-Cheon, Seoul 04702 (KR); KONG, Hyun-Seok, Seoul 03730 (KR); KIM, Jin-Man, Yongin-si Gyeonggi-do 16925 (KR)
(74) Representative: Thun, Clemens
(86) International application number: PCT/KR2017/009115
(87) International publication number: WO 2019/039620

(57) **Abstract**

The present invention relates to a composition for treating benign prostatic hyperplasia and a health functional food, containing an extract of *Aucklandiae Radix* and costunolide as active ingredients.

## Description

### Technical Field

The present invention relates to compositions and health functional foods containing an Aucklandiae Radix extract and costunolide as active ingredients for treatment of benign prostatic hyperplasia.

### Background Art

Aucklandiae Radix is the root of *Aucklandia lappa* Decne, a perennial herb of the family Asteraceae. Aucklandiae Radix (is described in herb medicines listed in the Korean Herbal Pharmacopoeia (KHP), and also described in oriental medical books, such as *Donguibogam, Bangyakhappyeon,* and *Bencaogangmu.*

The currently distributed Aucklandiae Radix is a cultivation product of A. *lappa,* which is not grown in Korea but is cultivated in Yunnan, Guangxi, and Sichuan in China. Aucklandiae Radix is distributed under name of Aucklandiae lappa radix or Saussurea radix depending on the cultivation region, but these are merely different names of Aucklandiae Radix and are originated from the identical *A. lappa.* In Korea, the roots of *Inula helenium* L. (having the same plant name as Aucklandiae Radix)of the family Asteraceae are called Helenii Radix, and *Inula helenium* L. is listed separately from *A. lappa* in the Korean Pharmacopoeia. Helenii Radix has been used as a substitute for Aucklandiae Radix, but is known to lack marketability.

The roots of *Vladimiria souliei* (Franch) Ling, which is another plant of the family Asteraceae, are also distributed under the name of Vladimiriae Radix in Chinese medicinal stuffs markets, and the roots of *Aristolochia contorta* Bunge of the family Aristolochia are also distributed under the name of Aristolochia Radix as a mixed product or a fake product of Aucklandiae Radix. However, these are banned in Korea due to differences in the composition and contents of bioactive compounds from Aucklandiae Radix.

The term "Aucklandiae Radix" encompasses Aucklandiae lappa radix, Helenii Radix, Vladimiriae Radix, and the like in the present invention including claims.

Aucklandiae Radix contains 0.3-2.78% of essential oil, and is an herb medicine containing 1.8% or more of a sum of costunolide (C₁₅H₂₀O₂: 232.32) and dehydrocostus lactone (C₁₅H₁₈O₂: 230.30) as main components. Aucklandiae Radix has aromatic stomachic, diuretic, apophlegmatic, and anthelmintic actions, and thus has been used for gastrointestinal disorders, such as indigestion, vomiting, vomiting and diarrhea, and diarrhea.

Especially, costunolide, which is one of the main components, belongs to terpene-based sesquiterpene lactones, and has been traditionally used as various therapeutic materials, such as antiinflammatory, antibacterial, and antiviral materials. Costunolide acts a strong antioxidant action of preventing vascular oxidation by blocking the activity of nitric oxide synthase. Costunolide is a substance that a plant produces to protect itself from oxidation stress, such as ultraviolet light or harmful insect damage, and also costunolide seems to perform similar actions in the human body. It has been recently reported that costunolide also has anticancer effects of preventing growth, angiogenesis, and metastasis of various cancer cell lines.

The prostate is a gland organ that is 4 x 3 x 3 cm³ in size and about 20 ml in volume, has a form in which fibromuscular tissue surrounds glandular tissue, and is located below the male bladder. The prostate grows 1.6 g every year to an adult size until the late 20s after the second sexual aging, and grows 0.4 g every year from the late 30s.

Benign prostatic hyperplasia is a disease that causes lower urinary tract obstruction or the like, accompanied by nodular hyperplasia and hypertrophy of prostate tissue due to increasing levels of dihydrotestosterone (DHT) together with relatively decreasing levels of testosterone in the body after the middle age, and 5-alpha reductase plays an important role in increasing DHT in the prostate tissue.

Endogenously, the apoptosis inhibiting gene survivin has been reported to increase and bcl-1 is known to be increasingly expressed in the prostate tissue in prostate cancer and benign prostatic hyperplasia, and therefore, through such a pathological mechanism, the prostate tissue overgrows, clinically resulting in various urination-related symptoms, such as urinary obstruction, a feeling of residual urine, urinary instability, difficulty in urination, and dysuria, and in severe cases, causing complications, such as calculosis, renal failure, hematuria, and infection.

Especially, benign prostatic hyperplasia has a great influence on the quality of life of the male population with the increase of the elderly population. In recent years, with the improvement of economic and social conditions, the attitude to the importance of benign prostatic hyperplasia is changing and the markets of therapeutics therefor are also expanding.

Until now, alpha adrenergic receptor blockers that perform symptom amelioration including urination improvement, 5-alpha reductase inhibitors that lower DHT levels, and some complementary and alternative therapies have been mainly used for benign prostatic hyperplasia, but the curative options were limited.

The alpha adrenergic receptor blockers include terazosin, doxazosin, alfuzosin, tamsulosin, and the like, and the 5-alpha reductase inhibitors include finasteride, dutasteride, and the like. Despite therapeutic benefits, these medicines have significant restrictions in their use due to the inherent adverse reactions thereof. For instance, the alpha adrenergic receptor blockers cause orthostatic hypertension and cardiovascular side effects, and the 5-alpha reductase inhibitors cause sides effects, such as decrease of sexual desire. For these reasons, complementary and alternative medicines have been proposed as alternatives, and saw palmetto or the like is frequently used. However, these medicines have limited effects, for example, the related academies in Korea suggested on the basis of oversea research results that such medicines are negative about benign prostatic hyperplasia relief effects, and therefore, there is a need for new therapeutically effective substances.

Up to now, nothing is known about the effects of the Aucklandiae Radix extract, which contains costunolide as an active ingredient and is noted by the present inventors, on the treatment of benign prostatic hyperplasia. The present inventors verified through cell tests and prostate-induced animal tests that the above ingredient has no hepatotoxicity and cytotoxicity but enhances immunity and decreases the prostate weight and volume, alleviating thickened prostate, and thus completed the present invention.

### Detailed Description of the Invention

### Technical Problem

The present invention has been made in view of the above-mentioned problems, and an aspect of the present invention is to provide a composition capable of being applied to benign prostatic hyperplasia by verifying that Aucklandiae Radix extract has an effect on benign prostatic hyperplasia.

### Technical Solution

In accordance with an aspect of the present invention, there is provided a composition containing an Aucklandiae Radix extract as an active ingredient for treatment and prevention of benign prostatic hyperplasia.

The Aucklandiae Radix extract may be obtained using any one of ethanol, methanol, and water as a solvent.

In accordance with another aspect of the present invention, there is provided a composition containing costunolide as an active ingredient for treatment and prevention of benign prostatic hyperplasia.

In accordance with still another aspect of the present invention, there is provided a health functional food containing the Aucklandiae Radix extract or costunolide for alleviation of benign prostatic hyperplasia.

### Advantageous Effects

As set forth below, the Aucklandiae Radix extract according to the present invention has an effect on benign prostatic hyperplasia.

### Brief Description of the Drawings

FIG. 1 shows the evaluation results of NO production rate and antiinflammatory effect according to the treatment with a 100% methanol extract of Aucklandiae Radix, a 30% ethanol extract of Aucklandiae Radix, a 70% ethanol extract of Aucklandiae Radix, and costunolide.
FIGS. 2a, 2b, and 2c show the measurement results of levels of inflammatory cytokines (IL-1β, IL-6, TNF-α) according to the treatment with a 30% ethanol extract of Aucklandiae Radix, a 70% ethanol extract of Aucklandiae Radix, and costunolide.
FIG. 3 shows benign prostatic hyperplasia alleviation effect according to the treatment with a 100% methanol extract of Aucklandiae Radix, a 30% ethanol extract of Aucklandiae Radix, a 70% ethanol extract of Aucklandiae Radix, and costunolide.
FIGS. 4 and 5 show the results of prostate pathology biopsy according to the Aucklandiae Radix extracts and costunolide.

### Mode for Carrying Out the Invention

Hereinafter, the present invention will be described in detail with reference to examples. The examples are provided merely to help with the understanding of the present invention, and the scope of the present invention is not limited to the examples.

### <1. Manufacture of Aucklandiae Radix extracts>

Aucklandiae Radix used in the present tests was purchased from Hunjin Pharmacy.

The purchased Aucklandiae Radix was subjected to extraction a total of twice using a reflux extractor (COSMOS660/80L, Misung Scientific. co. Ltd., Yangju, Kyunggido, Korea), and then the extracts were used in mixture. A solvent was added at 10 times the weight of the sample at the first extraction and added at 7 times the weight of the sample at the second extraction, followed by heat extraction for 3 hours each time of extraction.

The extracts were filtered (Whatman No 2, Maidstone, England), lyophilized (PVTFD10R, Ilsinb iobase, Dounducheon, Gyeonggido, Korea), milled (Cyclotec 1093 Sample Mill, Tecator Co., Sweden), and then stored frozen at -20°C before use.

As the solvent, 100% methanol, 70% ethanol, or 30% ethanol was used, and the extraction conditions were identical therefor.

### <2. HPLC analysis of Aucklandiae Radix extract>

The Aucklandiae Radix extracts were weighed 10 mg, respectively, and shaken to dissolve in methanol for 30 minutes or longer. The undissolved components were removed through a membrane filter (0.45 µm), and only the clear solution was subjected to HPLC analysis in the above conditions to obtain chromatograms, and the obtained results were summarized into a table (Table 1). The approximate concentrations were calculated by inserting each peak area from the chromatograms into the calibration equation, and the results verified that compared with the 30% ethanol extract, the 70% ethanol extract contained costunolide by about 1.8-fold and dehydrocostus lactone by about 1.6-fold.

**[Table 1]**

| EtOH extract | Concentration (ug/mL) | |
|---|---|---|
| | Costunolide | Dehydrocostuslactone |
| a) 30% extract | 80.0 (100%) | 106.4 (100%) |
| b) 70% extract | 145.4 (182%) | 165.1 (155%) |

### <3. Evaluation of antiinflammatory effect of Aucklandiae Radix extracts and costunolide>

### 1) Culture of macrophages

RAW 264.7 cells, which were macrophages used in the present tests, were available from American Type Culture Collection (ATCC). These cells were cultured in CO₂ incubator (Formascientific, Inc.) using DMEM containing 10% fetal bovine serum (FBS). The differentiation of cells was observed through microscopic observation, and then the cells were used in the tests.

### 2) Evaluation of nitric oxide (NO) change and antiinflammatory effect

To induce the activation of macrophages, RAW 264.7 cells were treated with 200 ng/Mℓ lipopolysaccharide (LPS) while simultaneously treated with various concentrations (µg/Mℓ) of a 100% methanol extract of Aucklandiae Radix (expressed by MeOH 100%), a 30% ethanol extract of Aucklandiae Radix (expressed by EtOH 30%), and a 70% ethanol extract of Aucklandiae Radix (expressed by EtOH 70%), and costunolide, and then the cells were cultured in a 37°C, 5% CO₂ incubator for 18 hours. After 18 hours, 100 µℓ of the supernatant was transferred to a new plate for each group, and 100 µℓ of a solution, in which Griess reagent A (2% sulfanilamide in 5% phosphoric acid) and Greiss reagent B (0.2% naphthylethylenediamine dihydrochloride) were mixed at a rate of 1:1, was added thereto. Thereafter, the absorbance was measured at 540 nm.

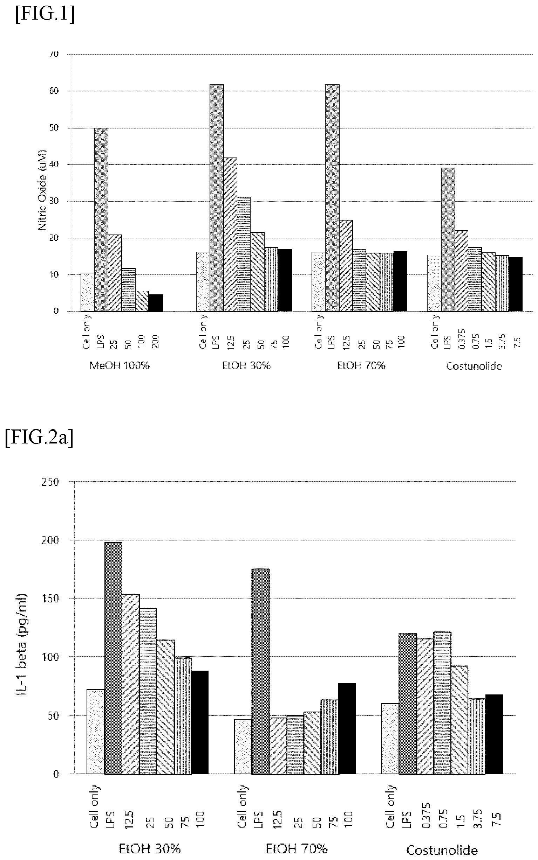

As a test result, the amount of NO was increased in the cell only treatment groups as the control groups rather than the LPS alone treatment groups, and the amount of NO was dose-dependent and was significantly decreased in all the groups treated with the Aucklandiae Radix extracts and costunolide (FIG. 1). It can be verified from these results that the Aucklandiae Radix extracts and costunolide had an anti-inflammatory effect of preventing tissue damage by suppressing excessive inflammation of cells.

### 3) Evaluation of inflammatory cytokine changes and antiinflammatory effect

RAW 264.7 cells were treated with 200 ng/Mℓ LPS while simultaneously treated with various concentrations (µg/Mℓ) of Aucklandiae Radix extracts (EtOH 30% and 70%) and costunolide, and then the cells were cultured in a 37°C, 5% CO₂ incubator for 18 hours. After 18 hours, ELISA analysis kits (eBioScience 88-7013, 88-7064 USA, BD 555268 USA) were used to check the levels of the inflammatory cytokines IL-1β, IL-6, and TNF-α in the supernatant of each group.

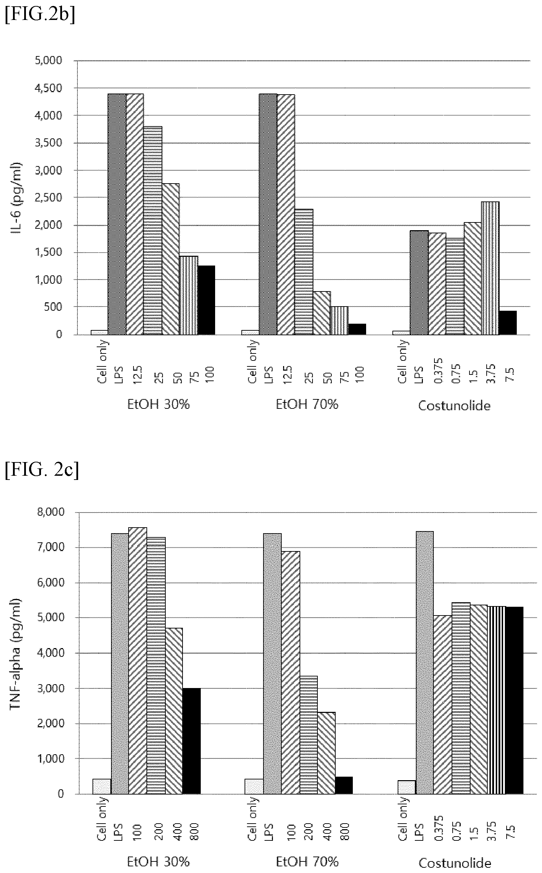

FIG. 2 shows the measurement results through the ELISA analysis kit of the levels of the inflammatory cytokines IL-1β (FIG. 2a), IL-6 (FIG. 2b), and TNF-α (FIG. 2c) in the cultures of macrophages activated by LPS. When compared with the cells treated with only LPS, the cells treated with the Aucklandiae Radix extracts and costunolide showed decreases in the secretion levels of the inflammatory cytokines, indicating that the Aucklandiae Radix extracts and costunolide had an antiinflammatory effect.

### <4. Evaluation of effect of Aucklandiae Radix extract on alleviation of benign prostatic hyperplasia>

### 1) Construction of benign prostatic hyperplasia animal models

After 6-week-old male Wistar rats (Samtako, Korea) were obtained and acclimated for one week, six animals constituted each test group when each animal weighed 340-350 g.

As for the test configuration, the animals were divided into groups without testicle removal and groups undergoing 4-hour recovery after testicle removal, and each group was subcutaneously injected with 5 mg/kg testosterone to induce benign prostatic hyperplasia for 8 weeks (BPH induced group). Test groups were constructed by oral administration of Aucklandiae Radix extracts and costunolide at different concentrations (mg/kg) once a day, seven times a week, for 8 weeks. Positive control groups were used by oral administration of finasteride, which has been used as a medicine for benign prostatic hyperplasia.

### 2) Changes in prostate weight and volume

After the completion of the test, the rats of all the test groups were euthanized, and then prostate tissue and main organs were extracted, and weights and volumes thereof were measured using an electronic scale for animal weights and a caliper (mm³) (Table 2).

The results verified that compared with the normal control groups, the BPH induced groups showed significantly increased weights and volumes, indicating the introduction of benign prostatic hyperplasia.

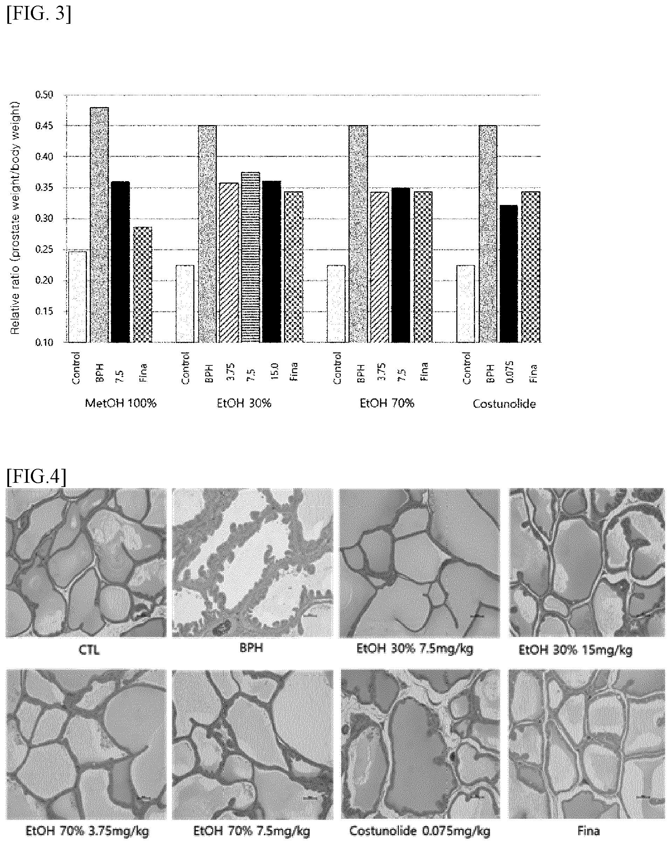

The prostate weights and volumes of the Aucklandiae Radix extract administration groups were all decreased to similar values to those of finasteride (BPH medicine) administration groups (Fina), and especially, the costunolide administration groups (0.075 µg/Mℓ) showed a significant reduction effect compared with the finasteride administration groups (FIG. 3).

**[Table 2]**

| | | | | | | | |
|---|---|---|---|---|---|---|---|
| (weight: g, volume: mm³) | | | | | | | |

| Administered material | Test group | Body weight (a) | Prostate weight (b) | Ratio (b/a * 100) | Prostate volume | Liver weight | Spleen weight |
|---|---|---|---|---|---|---|---|
| MeOH | control | 391 | 0.97 | 0.25 | 2.353 | - | 0.71 |
| | BPH | 384 | 1.82 | 0.48 | 6.145 | - | 0.64 |
| | 7.5mg/kg | 377 | 1.37 | 0.36 | 3.530 | - | 0.65 |
| | Fina | 379 | 1.23 | 0.29 | 2.322 | - | 0.60 |
| EtOH 30 | control | 489 | 1.09 | 0.22 | 2.952 | 11.28 | 0.72 |
| | BPH | 414 | 1.87 | 0.45 | 6.079 | 9.57 | 0.62 |
| | 3.75mg/kg | 439 | 157 | 0.36 | 4.820 | 9.55 | 067 |
| | 7.5mg/kg | 419 | 1.57 | 0.38 | 4.864 | 10.14 | 0.66 |
| | 15mg/kg | 411 | 1.47 | 0.36 | 4.443 | 9.86 | 0.72 |
| | Fina | 415 | 1.42 | 0.34 | 4.255 | 9.46 | 0.70 |
| EtOH 70 | control | 489 | 1.09 | 0.22 | 2.952 | 11.28 | 0.72 |
| | BPH | 414 | 1.87 | 0.45 | 6.079 | 9.57 | 0.62 |
| | 3.75mg/kg | 420 | 1.44 | 0.34 | 4.322 | 10.07 | 0.65 |
| | 7.5mg/kg | 420 | 1.46 | 0.35 | 4.394 | 9.33 | 0.62 |
| | Fina | 415 | 1.42 | 0.34 | 4.255 | 9.46 | 0.70 |
| Costunolide | control | 489 | 1.09 | 0.22 | 2.952 | 11.28 | 0.72 |
| | BPH | 414 | 1.87 | 0.45 | 6.079 | 9.57 | 0.62 |
| | 0.075mg/kg | 433 | 1.40 | 0.32 | 4.126 | 10.72 | 0.62 |
| | Fina | 415 | 1.42 | 0.34 | 4.255 | 9.46 | 0.70 |

### 3) Measurement of hepatotoxicity and kidney function change

For analysis of biochemical markers in serum, blood was obtained through the abdominal vein from rats of all the groups at necropsy. The blood was coagulated for about 30 minutes, and then centrifuged for 5 minutes at 10,000 rpm to separate serum. After serum separation, a biochemistry analyzer (AU480, Beckman Coulter, USA) was used to check liver function (AST, ALP), lipoprotein (total cholesterol (T-CHO)), HDL cholesterol(HDL-C), LDL cholesterol(LDL-C)), and kidney function (creatine) levels (Table 3). The results verified that all the groups administered with the extracts and costunolide showed no significant changes, indicating no hepatoxicity and kidney toxicity.

**[Table 3]**

| Administered material | Test group | AST (U/L) | ALP (U/L) | T-CHO (mg/dL) | HDL (mg/dL) | LDL (mg/dL) | CRE (mg/dL) |
|---|---|---|---|---|---|---|---|
| MeOH | control | 77.20 | 32.60 | 59.20 | 19.40 | 16.00 | 0.40 |
| | BPH | 107.60 | 43.80 | 60.20 | 19.60 | 14.40 | 0.30 |
| | 7.5mg/kg | 105.80 | 51.40 | 67.60 | 21.60 | 17.20 | 0.28 |
| | Fina | 88.20 | 37.20 | 59.00 | 19.20 | 13.20 | 0.30 |
| EtOH 30 | control | 96.00 | 91.50 | 93.33 | 60.00 | 12.67 | 0.37 |
| | BPH | 125.17 | 85.67 | 88.17 | 56.17 | 13.50 | 0.35 |
| | 3.75mg/kg | 89.00 | 71.33 | 66.50 | 43.00 | 10.67 | 0.30 |
| | 7.5mg/kg | 121.67 | 86.33 | 79.50 | 50.00 | 12.67 | 0.35 |
| | 15mg/kg | 145.00 | 70.83 | 86.33 | 55.00 | 13.00 | 032 |
| | Fina | 80.67 | 81.50 | 70.83 | 43.83 | 13.67 | 0.27 |
| EtOH 70 | control | 96.00 | 91.50 | 93.33 | 60.00 | 12.67 | 0.37 |
| | BPH | 125.17 | 85.67 | 88.17 | 56.17 | 13.50 | 0.35 |
| | 3.75mg/kg | 90.33 | 67.67 | 73.00 | 47.17 | 12.50 | 0.28 |
| | 7.5mg/kg | 70,50 | 64.33 | 71.83 | 47.67 | 12.00 | 0.30 |
| | Fina | 80.67 | 81.50 | 70.83 | 43.83 | 13.67 | 0.27 |
| Costunolide | control | 96.00 | 91.50 | 93.33 | 60.00 | 12.67 | 0.37 |
| | BPH | 125.17 | 85.67 | 88.17 | 56.17 | 13.50 | 0.35 |
| | 0.075mg/kg | 78.33 | 61.17 | 79.67 | 51.50 | 13.17 | 0.27 |
| | Fina | 80.67 | 81.50 | 70.83 | 43.83 | 1367 | 0.27 |

### 4) Prostate pathology biopsy

After the completion of the test, the prostate tissue extracted from each group was fixed with 10% neutral formalin and stored. The tissue of each group was processed into paraffin blocks, and slides for microscopic inspection of tissue were manufactured and stained with hematoxylin & eosin (H&E). The microscopic inspection of tissue was conducted through a microscope (Olympus, Tokyo, Japan) at a magnification of X200.

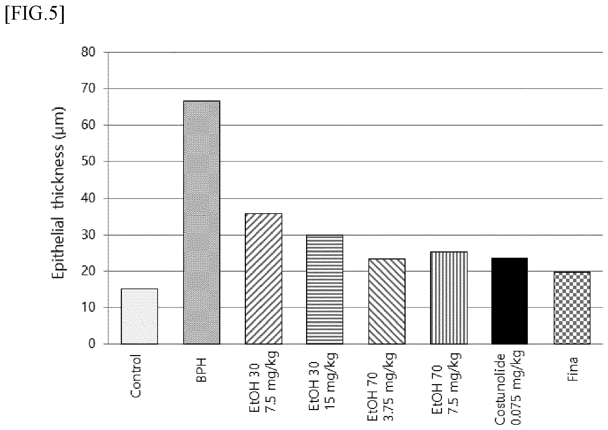

It can be seen that compared with the normal control groups, the benign prostatic hyperplasia induced groups showed atrophy and hypertrophy of epithelial cells of prostate vesicles and overall thickening of the basement membrane. The test groups administered with Aucklandiae Radix extracts and costunolide showed alveolar glands maintained in a round shape without atrophy similar to the normal control group, and showed significantly reduced endothelial thicknesses compared with the benign prostatic hyperplasia induced group (FIGS. 4 and 5).

### <5. Evaluation of toxicity of Aucklandiae Radix extracts >

For investigation of a safety area of the Aucklandiae Radix extracts, the acute toxicity test and MTD toxicity test were carried out.

For the acute toxicity test, 7-week-old healthy male and female SD rats were divided into one normal control group and two drug administration groups (two groups orally administered with the Aucklandiae Radix extract at a single dose of 1,000 mg and 2,000 mg per kg of body weight), and five animals were taken for each group, and then tested. As a test result, there were no significant differences in body weight, feed intake, and the like between the normal control group and the drug administration groups, and no toxicity-related clinical signs were observed (Table 4: Acute toxicity test results).

**[Table 4]**

| Group | N | **Male** | | | | **Female** | | | |
|---|---|---|---|---|---|---|---|---|---|
| | | B.W. (g) | | | c.s.* | B.W. (g) | | | c.s.* |
| | | Day 1 | Day 7 | Day 14 | Day 14 | Day 1 | Day 7 | Day 14 | Day 14 |
| Control | 5 | 210±9 | 240±13 | 283±18 | No sign | 178±11 | 193±8 | 216±11 | No sign |
| 1000mg/kg | 5 | 208±10 | 247±10 | 285±12 | No sign | 177±10 | 195±10 | 214±15 | No sign |
| 2000mg/kg | 5 | 207±11 | 242±13 | 285±16 | No sign | 175±9 | 193±6 | 208±9 | No sign |

| | | | | | | | | | |
|---|---|---|---|---|---|---|---|---|---|
| - Body weight, feed intake, and clinical signs: no significant differences between normal control group and Aucklandiae Radix extract administration groups - C.S.* : Clinical sign | | | | | | | | | |

For the MTD toxicity test, 7-week-old healthy male and female SD rats were divided into two medicine administration groups (two groups orally administered with 30% and 70% ethanol extracts of the Aucklandiae Radix extract for 5 days), and three animals were taken for each group, and then tested. As a test result, there were no significant differences in body weight, feed intake, and the like between the medicine administration groups, and no toxicity-related signs were observed in clinical testing and necropsy (Table 5: MTD toxicity test results).

**[Table 5]**

| Group | Male | N | B.W.(g) | | | C.S.* | N.F.** |
|---|---|---|---|---|---|---|---|
| | (mg/kg/day) | | Day 1 | Day 3 | Day 5 | Day 1 - 5 | Day 1 - 5 |
| KSB30% EtOH | 1000 x 5days | 3 | 222±7 | 232±7 | 248±6 | No change | No change |
| KSB70% EtOH | 1000 x 5days | 3 | 220±4 | 231±4 | 244±2 | No change | No change |

| | | | | | | | |
|---|---|---|---|---|---|---|---|
| - Body weight, feed intake, and clinical signs: no significant differences between two groups. - C.S.* : Clinical Sign, NF** : Necropsy Findings | | | | | | | |

It can be seen from the above results that Aucklandiae Radix extracts can alleviate the thickened prostate by increasing immune-related antiinflammatory responses and reducing prostate weight and volume without general toxicity.

## Claims

1. A composition containing an Aucklandiae Radix extract as an active ingredient for treatment and prevention of benign prostatic hyperplasia.

2. The composition of claim 1, wherein the Aucklandiae Radix extract is obtained using any one of ethanol, methanol, and water as a solvent.

3. A composition containing costunolide as an active ingredient for treatment and prevention of benign prostatic hyperplasia.

4. A health functional food containing the ingredient of any one of claims 1 to 3 for alleviation of benign prostatic hyperplasia
